(19) Europäisches Patentamt
European Patent Office
Office européen des brevets

(11) Veröffentlichungsnummer: 0 138 757
B1

(12) # EUROPÄISCHE PATENTSCHRIFT

(45) Veröffentlichungstag der Patentschrift: **18.10.89**

(51) Int. Cl.⁴: **C 07 C 103/375**, C 07 C 102/00, C 07 C 149/42, C 07 D 213/64, A 01 N 37/22, A 01 N 43/40

(21) Anmeldenummer: **84810411.3**

(22) Anmeldetag: **20.08.84**

(54) **Oxalsäureanilide.**

(30) Priorität: **26.08.83 CH 4670/83**
**27.03.84 CH 1525/84**

(43) Veröffentlichungstag der Anmeldung:
**24.04.85 Patentblatt 85/17**

(45) Bekanntmachung des Hinweises auf die Patenterteilung:
**18.10.89 Patentblatt 89/42**

(84) Benannte Vertragsstaaten:
**AT BE CH DE FR IT LI NL**

(56) Entgegenhaltungen:
**DE-A-1 907 403**
**DE-A-2 139 781**
**DE-B-1 568 351**
**DE-B-1 911 835**
**GB-A-1 177 095**
**US-A-3 906 033**

(73) Patentinhaber: **CIBA-GEIGY AG, Klybeckstrasse 141, CH-4002 Basel (CH)**

(72) Erfinder: **Böger, Manfred, Wilhelm Glock-Strasse 14, D-7858 Weil am Rhein 5 (DE)**
Erfinder: **Drabek, Jozef, Dr., Benkenstrasse 12, CH-4104 Oberwil (CH)**
Erfinder: **Neumann, Rainer, Dr., Rooseveltstrasse 4, CH-4102 Binningen (CH)**

**EP 0 138 757 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Oxalsäuredianilide, diese Verbindungen enthaltende Schädlingsbekämpfungsmittel, Verfahren zu ihrer Herstellung und ihre Verwendung als Pestizide.

Ein Gegenstand der vorliegenden Erfindung ist somit ein Mittel zur Bekämpfung von Schädlingen, insbesondere von Insekten und Vertretern der Ordnung Akarina, das als aktive Komponente mindestens eine Verbindung der Formel I

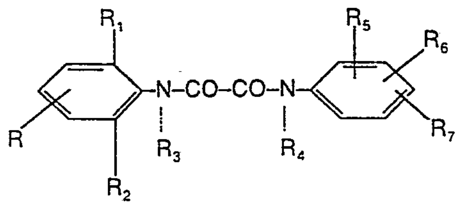 (I),

worin

R Wasserstoff, Halogen, $C_1$–$C_4$-Alkyl, Methoxy, Äthoxy, $C_1$–$C_2$-Fluoralkoxy mit 1 bis 5 Fluoratomen;

$R_1$ Wasserstoff, Halogen, Methyl, Methoxy, Äthoxy, $C_1$–$C_2$-Fluoralkoxy mit 1 bis 5 Fluoratomen, $C_1$–$C_3$-Alkylthio oder Cyano;

$R_2$ Wasserstoff, Halogen, Methyl oder Methoxy;

$R_3$ und $R_4$ unabhängig voneinander Wasserstoff oder Methyl;

$R_5$ Wasserstoff, Halogen, Methyl, Acetyl oder Trifluormethyl;

$R_6$ Wasserstoff, Halogen, Methyl, Trifluormethyl oder Carbalkoxy mit 1 bis 4 Kohlenstoffatomen im Alkylteil; und

$R_7$ Wasserstoff, $C_1$–$C_3$-Fluoralkyl mit 1 bis 7 Fluoratomen, $C_1$–$C_3$-Alkoxy, $C_1$–$C_3$-Halogenalkoxy mit 1 bis 7 Halogenatomen oder den Rest

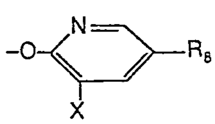 ,

wobei $R_8$ für Wasserstoff, Chlor, Trifluormethyl oder einen perhalogenierten Äthylrest und X für Wasserstoff oder Chlor stehen, oder den Rest

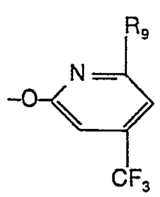 ,

wobei $R_9$ für Wasserstoff, Chlor, Brom, Methoxy oder Äthoxy steht, oder den Rest

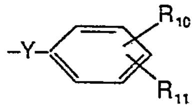

wobei $R_{10}$ und $R_{11}$ unabhängig voneinander für Wasserstoff, Halogen, Methyl, Trifluormethoxy oder Trifluormethyl und Y für ein Sauerstoff- oder Schwefelatom stehen, oder den Rest

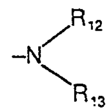 ,

wobei $R_{12}$ und $R_{13}$ für $C_1$–$C_5$-Alkyl stehen; bedeuten, mit der Massgabe dass $R_5$, $R_6$ und $R_7$ nicht gleichzeitig Wasserstoff bedeuten, zusammen mit geeigneten Trägern und anderen Zuschlagstoffen enthält.

Ein weiterer Gegenstand der Erfindung ist die Verwendung der vorstehend genannten Verbindungen der Formel I zur Bekämpfung von Insekten.

Ein weiterer Gegenstand der Erfindung sind neuartige Verbindungen der Formel Ia

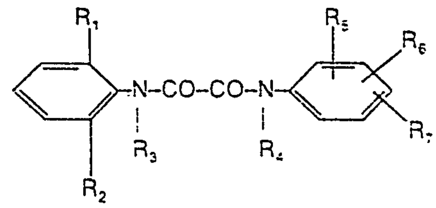 (Ia),

worin

R Wasserstoff, Fluor, Chlor oder Methoxy;

$R_2$ Wasserstoff, Fluor, Chlor, Brom oder Methoxy;

$R_3$ und $R_4$ unabhängig voneinander Wasserstoff oder Methyl;

$R_5$ und $R_6$ unabhängig voneinander Wasserstoff, Chlor oder Methyl;

$R_7$ Trifluormethoxy, dem Rest $-O-CF_2-CHF_2$ oder den Rest

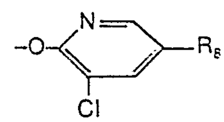 ,

wobei $R_8$ für Trifluormethyl oder einen der Reste $-CF_2-CF_2Cl$, $-CF_2-CF\ Cl_2$, $-CCl_2-CCl_3$, $-CF_2-CCl_3$ oder $-CF_2-CF_3$ steht, oder den Rest

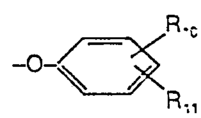 ,

wobei $R_{10}$ und $R_{11}$ unabhängig voneinander für Wasserstoff oder Chlor stehen, oder den Rest

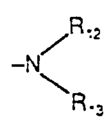 ,

wobei $R_{12}$ für Methyl und $R_{13}$ für $C_1$–$C_5$-Alkyl stehen;
bedeuten.

Wegen ihrer guten pestiziden Wirkung hervorzuheben sind diejenigen Verbindungen der Formeln I bzw. Ia, worin $R_7$ in 4-Stellung am Phenylring steht.

Weiterhin bevorzugte Verbindungen der Formeln I und Ia sind dadurch gekennzeichnet, dass

$R_5$ Wasserstoff bedeutet und $R_7$ in 5-Stellung am Phenylring steht;

dadurch gekennzeichnet, daß $R_6$ in 4-Stellung am Phenylring steht; oder dadurch gekennzeichnet, daß $R_5$ und $R_6$ in 3- bzw. 5-Stellung am Phenylring stehen.

Von besonderem Interesse wegen ihrer biologischen Wirkung sind solche Verbindungen der Formel I bzw. Ia, worin $R_7$ einen in 4-Stellung befindlichen Rest aus der Gruppe

$—O—CF_2CHF_2$ , 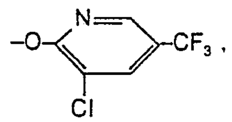 ,

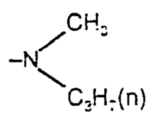 oder 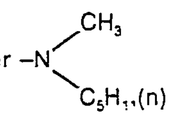

bedeutet.

Unter dem Begriff Alkyl sind geradkettige und verzweigte Alkylreste und je nach Zahl der angegebenen Kohlenstoffatome beispielsweise folgende Gruppen zu verstehen: Methyl, Äthyl, Propyl, Butyl und Pentyl, sowie ihre Isomere, wie z.B. Isopropyl, Isobutyl, tert.-Butyl, Isopentyl usw.

Unter dem Begriff Halogen im Rahmen der vorliegenden Erfindung ist Fluor, Chlor und Brom zu verstehen, vorzugsweise Fluor und Chlor.

Die Verbindung der Formel I können analog an sich bekannten Verfahren (vgl. deutsche Offenlegungsschrift Nr. 3 135 810) hergestellt werden.

Entsprechend können die erfindungsgemäßen neuen Verbindungen der Formel Ia erhalten werden, indem man

a) eine Verbindung der Formel II

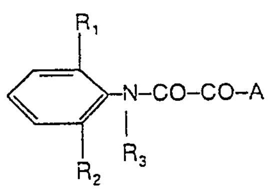 (II)

mit einer Verbindung der Formel III

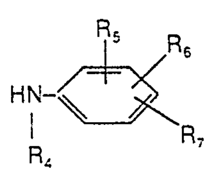 (III)

oder

b) eine Verbindung der Formel IV

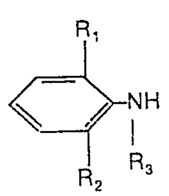 (IV)

mit einer Verbindung der Formel V

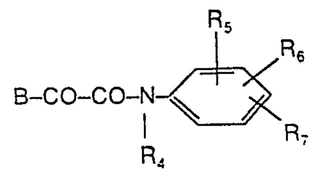 (V)

umsetzt, wobei in den Formeln II bis V die Reste $R_1$ bis $R_7$ die für Formel I a angegebenen Bedeutungen haben, A Methoxy, Äthoxy oder Halogen, vorzugsweise Chlor, und B Methoxy oder Äthoxy bedeuten.

Die vorerwähnten Verfahren a) und b) werden vorzugsweise unter normalem Druck und durch direktes Erhitzen bzw. Verschmelzen der Reaktionspartner durchgeführt, wobei die Temperatur zwischen etwa 40° und 220 °C, vorzugsweise zwischen 50° und 200 °C, liegt. die Umsetzungen können aber auch in Gegenwart eines inerten, verhältnismäßig hoch siedenden Lösungs- oder Verdünnungsmittels durchgeführt werden, wobei die Umsetzungstemperatur zwischen etwa 80° und 200 °C, vorzugsweise bei Rückflußtemperatur des verwendeten Lösungsmittels, liegt. Als Lösungs- oder Verdünnungsmittel eignen sich z.B. ätherartige Verbindungen, wie Dibutyläther, Dioxan, Dimethoxyäthan, und verätherte Alkylenglykole; N,N-dialkylierte Carbonsäureamide; aliphatische, aromatische sowie halogenierte Kohlenwasserstoffe, insbesondere Toluol, Xylole, Chlorbenzol; Dimethylsulfoxid sowie Ketone, z.B. Methylisopropylketon und Methylisobutylketon.

Die Umsetzung kann durch Zusatz von Katalysatoren, z.B. Borsäure, beschleunigt werden. Wenn beim Verfahren a) als Ausgangsmaterial z.B. ein Anilidooxalylchlorid der Formel II eingesetzt wird, verwendet man im allgemeinen eines der bekannten basischen Kondensationsmittel, z.B. Dialkylamine, Triäthylamin, Pyridin, Cholin und anorganische Basen, wie Na-Carbonat und Na- oder K-Bicarbonat.

Die als Ausgangsmaterialien verwendeten Aniline der Formeln III und IV sowie die Anilidooxalylderivate der Formeln II und V sind bekannt oder können analog bekannten Arbeitsweisen erhalten werden. Die Anilidooxalsäureester der Formeln II und V sind z.B. durch Umsetzung von Oxalsäuredialkylestern mit den entsprechenden Anilinen der Formeln IV bzw. III zugänglich (vgl. deutsche Offenlegungsschrift Nr. 3 135 810).

Aniline der Formel III, worin $R_7$ den Rest

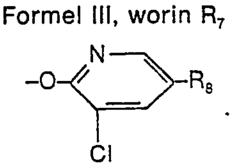

bedeutet, und deren Herstellung sind aus den deutschen Offenlegungsschriften Nr. 3241138 bzw. 3240975 bekannt.

Aus der französischen Patentanmeldung Nr. 2 504 915 und der deutschen Offenlegungsschrift Nr. 3135810 sind Oxalsäuredianilide mit unsymmetrischer Substitution in bezug auf die beiden Phenylreste bekannt. Die dort beschriebenen Verbindungen sollen als Stabilisierungsmittel für chlorierte Paraffine gegen Hitze und UV-Einwirkung bzw. zur Stabilisierung von Lacken und Lackierungen geeignet sein. Weiterhin sind aus den deutschen Offenlegungsschriften Nr. 2139781, 1907403 und 1568351, der deutschen Auslegeschrift Nr. 1911835, der britischen Patentschrift Nr. 1.177.095 und der US-Patentschrift 3.906.033 symmetrisch und unsymmetrisch substituierte Oxalsäuredianilide mit unterschiedlichen Substituenten als UV- und Wärme-Stabilisierungsmittel von polymeren organischen Materialien, z.B. Polyolefinen, bekannt. Demgegenüber wurde nun gefunden, daß die erfindungsgemäß vorgeschlagenen Oxalsäuredianilide der Formel I, die strukturspezifisch in erster Linie durch die Substituentenanordnung an den Phenylresten charakterisiert sind, überraschenderweise ausgezeichnete Wirksamkeit als Schädlingsbekämpfungsmittel, insbesondere im Pflanzenschutz, aufweisen. Ein besonderer Vorteil der erfindungsgemäß zu verwendenden Verbindungen der Formel I ergibt sich aus ihrer sehr geringen Warmblütertoxizität bei guter Pflanzenverträglichkeit. Besonders gute pestizide Wirksamkeit weisen die an sich neuartigen erfindungsgemäßen Verbindungen der Formel Ia auf.

Insbesondere eignen sich die Verbindungen der Formel I zur Bekämpfung von Insekten der Ordnungen: Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera, sowie von Vertretern der Ordnung Akarina der Familien: Ixodidae, Argasidae, Tetranchidae und Dermanyssidae.

Neben ihrer Wirkung gegenüber Fliegen, wie z.B. Musca domestica, und Mückenlarven können Verbindungen der Formel I auch zur Bekämpfung von pflanzenschädigenden Frassinsekten in Zier- und Nutzpflanzungen, insbesondere in Baumwollkulturen (z.B. Spodoptera littoralis und Heliothis virescens), sowie in Obst- und Gemüsekulturen (z.B. Laspeyresia pomonella, Leptinotarsa decemlineata und Epilachna varivestis) eingesetzt werden. Die Verbindungen der Formel I zeichnen sich durch eine ausgeprägte ovizide und vor allem larvizide Wirkung gegen Insekten, insbesondere Larven von fressenden Schadinsekten, aus. Werden Verbindungen der Formel I von adulten Insekten-Stadien mit dem Futter aufgenommen, so ist in vielen Fällen, insbesondere bei Coleopteren, wie z.B. Anthonomus grandis, eine verminderte Ei-Ablage und/oder reduzierte Schlupfrate festzustellen.

Die Verbindungen der Formel I können ferner zur Bekämpfung von Ektoparasiten, wie Lucilia sericata, an Haus- und Nutztieren eingesetzt werden, z.B. durch Tier-, Stall- und Weidebehandlung.

Die Verbindungen der Formel I eignen sich ferner zur Bekämpfung der folgenden Obst- und Gemüsekulturen befallenden Milbenspezies: Tetranychus urticae, Tetranychus cinnabarinus, Panonychus ulmi, Broybia rubrioculus, Panonychus citri, Eriophyes piri, Eriophyes ribis, Eriophyes vitis, Tarsonemus pallidus, Phyllocoptes vitis und Phyllocoptruta oleivora.

Die gute pestizide Wirkung der erfindungsgemäßen Verbindungen der Formel I entspricht einer Abtötungsrate (Mortalität) von mindestens 50–60% der erwähnten Schädlinge.

Die Wirkung der erfindungsgemäßen Verbindungen bzw. der sie enthaltenden Mittel läßt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindung der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren, wie Versprühen, Vernebeln, Verstäuben, Verstreuen oder Gießen, werden ebenso wie die Mittel den angestrebten Zielen und den gegebenen Verhältnissen entsprechend gewählt.

Die Formulierung, d.h. die den Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmoittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Äther und Ester, wie Äthanol, Äthylenglykol, Äthylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnußöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte adsorptive Granulatträger kommen poröse Typen, wie z.B. Bimsstein, Ziegelbruch, Sepiolit oder Bentonit, als nicht sorptive

Trägermaterialien z.B. Calcit oder Sand in Frage. Darüberhinaus kann eine Vielzahl von granulierten Materialen anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$–$C_{22}$), wie z.B. die Na- oder K-Salze oder Öl- oder Stearinsäure, oder von natürlichen Fettsäuregemischen, die z.B. aus Kokosnuß- oder Talgöl gewonnen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschließt, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2 Sulfonsäuregruppen und einen Fettsäurerest mit etwa 8–22 C-Atomen. Alkylarylsulfonate sind z.B. die Na-, Ca- oder Triäthanolaminsalze oder Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Äthylenoxid-Adduktes, in Frage.

Als nichtionisches Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:

«Mc Cutcheon's Detergents and Emulsifiers Annual» MC Publishing Corp., Rindgewood, New Jersey, 1979;

Dr. Helmut Stache «Tensid Taschenbuch», Carl Hanser Verlag München/Wien 1981.

Die pestiziden Zubereitungen enthalten in der Regel 0,1 bis 99%, insbesondere 0,1 bis 95%, Wirkstoff der Formel I oder Kombinationen dieser Wirkstoffe mit andern Insektiziden oder Akariziden, 1 bis 99,9% eines festen oder flüssigen Zusatzstoffes und 0 bis 25%, insbesondere 0,1 bis 20%, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Formulierungsbeispiele für Wirkstoffe der Formel I resp. Kombinationen dieser Wirkstoffe mit andern Insektiziden oder Akariziden (% = Gewichtsprozent)

| 1. Spritzpulver | a) | b) | c) |
|---|---|---|---|
| Wirkstoff oder Wirkstoffkombination | 25% | 50% | 75% |
| Na-Ligninsulfonat | 5% | 5% | – |
| Na-Laurylsulfat | 3% | – | 5% |
| Na-Diisobutylnaphthalinsulfonat | – | 6% | 10% |
| Octylphenolpolyäthylenglykoläther (7–8 Mol AeO) | – | 2% | – |
| Hochdisperse Kieselsäure | 5% | 10% | 10% |
| Kaolin | 62% | 27% | – |

Der Wirkstoff oder die Wirkstoffkombination wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

2. Emulsions-Konzentrat

| | |
|---|---|
| Wirkstoff oder Wirkstoffkombination | 10% |
| Octylphenolpolyäthylenglykoläther (4–5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| 3. Stäubemittel | a) | b) |
|---|---|---|
| Wirkstoff oder Wirkstoffkombination | 5% | 8% |
| Talkum | 95% | – |
| Kaolin | – | 92% |

Man erhält anwendungsfertige Stäubemittel, indem der Wirkstoff mit dem Träger vermischt und auf einer geeigneten Mühle vermahlen wird.

4. Extruder-Granulat

| | |
|---|---|
| Wirkstoff oder Wirkstoffkombination | 10% |
| Na-Ligninsulfonat | 2% |
| Carboxymethylcellulose | 1% |
| Kaolin | 87% |

Der Wirkstoff oder die Wirkstoffkombination wird mit den Zusatzstoffen vermischt, vermahlen und mit Wasser angefeuchtet. Dieses Gemisch wird extrudiert, granuliert und anschließend mit Lufstrom getrocknet.

5. Umhüllungs-Granulat

| | |
|---|---|
| Wirkstoff oder Wirkstoffkombination | 3% |
| Polyäthylenglykol (MG 200) | 3% |
| Kaolin | 94% |

Der fein gemahlene Wirkstoff oder die Wirkstoffkombination wird in einem Mischer auf das mit Polyäthylenglykol angefeuchtete Kaolin gleichmäßig aufgetragen. Auf diese Weise erhält man staubfreie Umhüllungs-Granulate.

6. Suspensions-Konzentrat

| | |
|---|---|
| Wirkstoff oder Wirkstoffkombination | 40 % |
| Äthylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2% |
| Silikonöl in Form einer 75%igen wäßrigen Emulsion | 0,8% |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 1

Herstellung von N-(2,6-Difluorphenyl)-N'-[4-(3-chlor-5-trifluormethyl-2-pyridyloxy)-3,5-dichlorphenyl]-oxalsäuredianilid:

Es werden 8,9 g 3-Chlor-5-trifluormethyl-2-pyridyl-2,6-dichlor-4-aminophenyläther und 5,4 g 2,6-Difluorphenylamino-oxo-esssigsäuremethylester zusammen mit 20 ml Chlorbenzol während 3 Stunden unter Rühren zum Sieden erwärmt (Badtemperatur ca. 150 °C). Nach dem Abkühlen wird das Reaktionsgemisch mit Hexan versetzt, der Rückstand abgenutscht und mit Hexan gewaschen. Nach dem Umkristallisieren aus Toluol erhält man die Titelverbindung der Formel

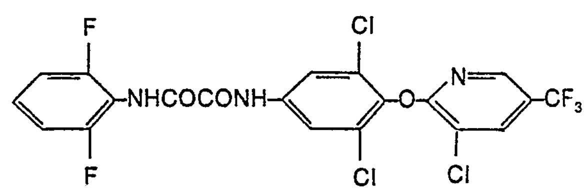

als weisses kristallines Produkt vom Schmelzpunkt 202–203 °C (Verbindung Nr. 1).

Analog den vorstehend beschriebenen Arbeitsweisen werden die folgenden Verbindungen der Formel I hergestellt:

| Verbindung Nr. | | Smp. [°C] |
|---|---|---|
| 2 | 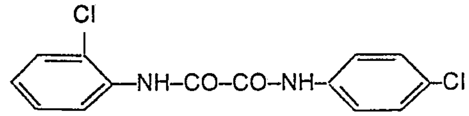 | 212–213 |
| 3 | 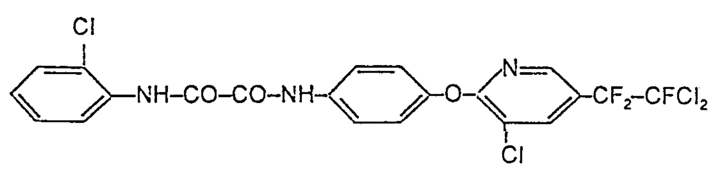 | 174–176 |

| Verbindung Nr. | | Smp. [°C] |
|---|---|---|
| 4 | 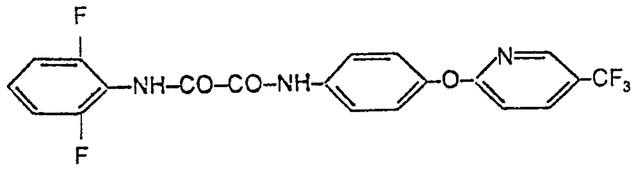 | 209–211 |
| 5 | 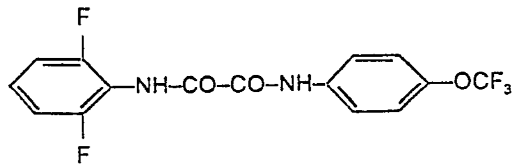 | 203–205 |
| 6 | 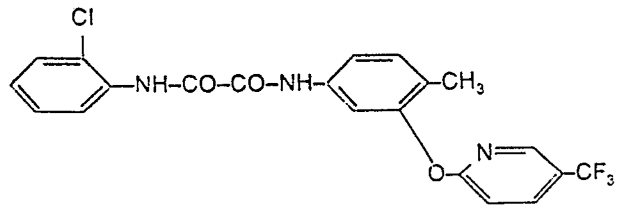 | 177–179 |
| 7 | 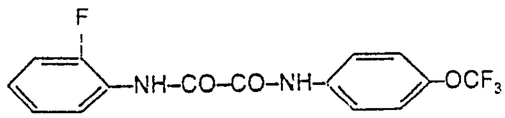 | 183–185 |
| 8 | 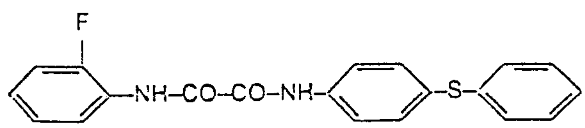 | 166–168 |
| 9 | 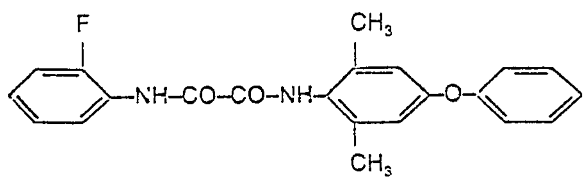 | 144–146 |
| 10 | 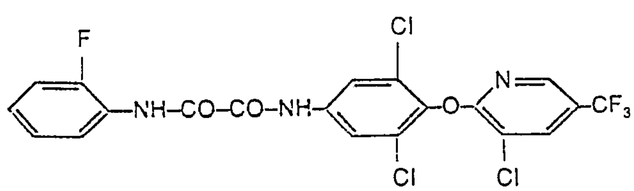 | 165–167 |
| 11 | 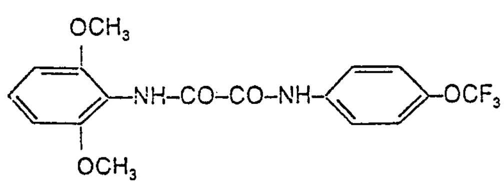 | 186–188 |
| 12 | 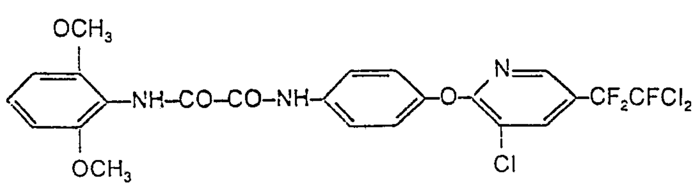 | 204–206 |

| Verbindung Nr. | | Smp. [°C] |
|---|---|---|
| 13 | 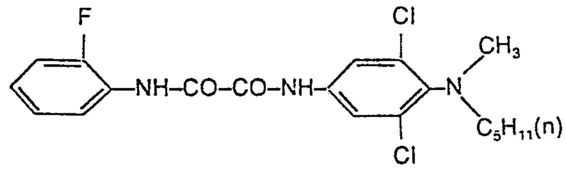 | 74–76 |
| 14 | 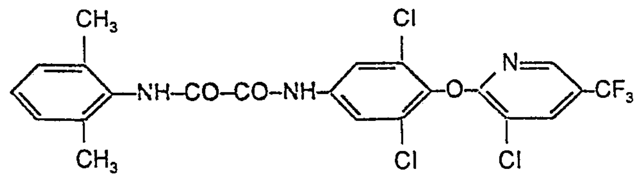 | 163–165 |
| 15 | 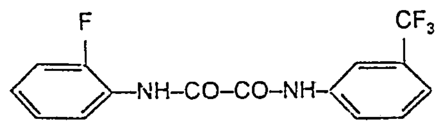 | 140–142 |
| 16 | 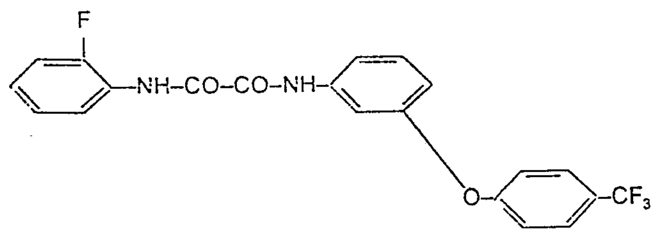 | 137–140 |
| 17 | 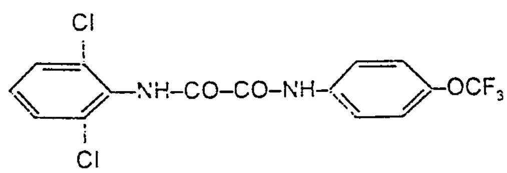 | 211–212 |
| 18 | 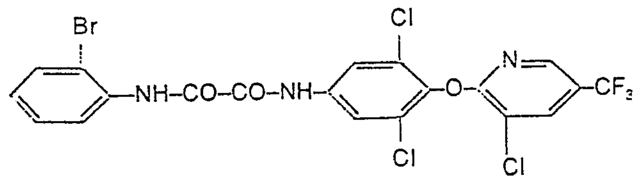 | 155–157 |
| 19 | 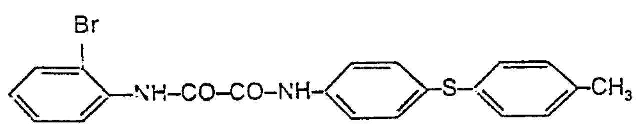 | 167–169 |
| 20 | 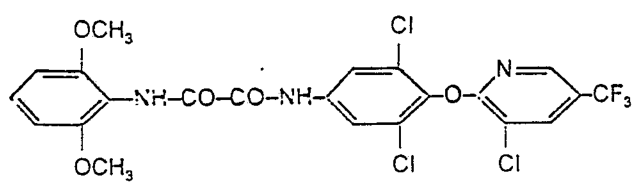 | 205–207 |
| 21 | 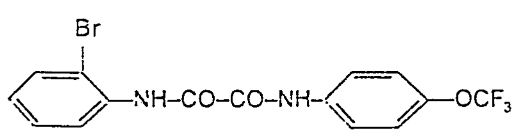 | 172–173 |

| Verbindung Nr. | | Smp. [°C] |
|---|---|---|
| 22 | 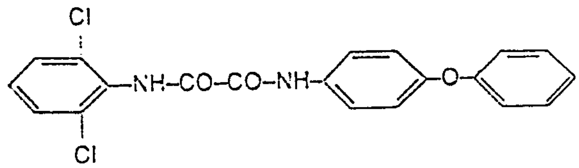 | 202–204 |
| 23 | 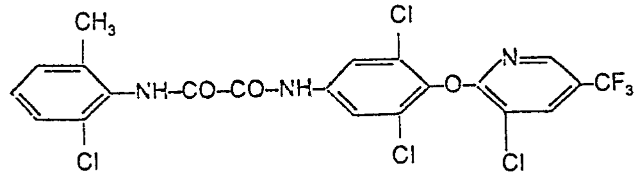 | 197–199 |
| 24 | 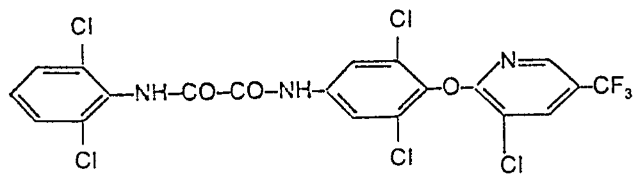 | 225–227 |
| 25 | 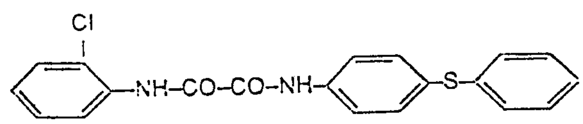 | 174–175 |
| 26 | 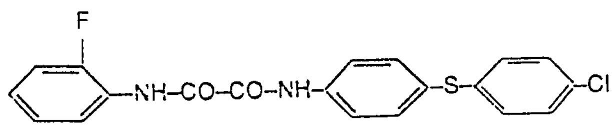 | 169–170 |
| 27 | 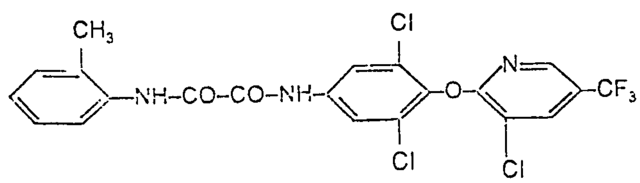 | 139–141 |
| 28 | 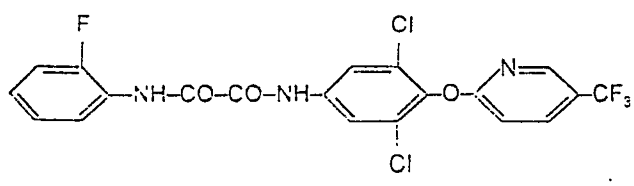 | 162–164 |
| 29 | 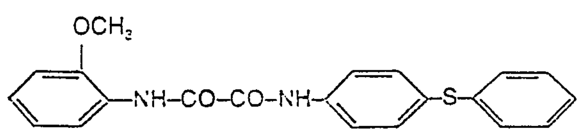 | 139–141 |
| 30 | 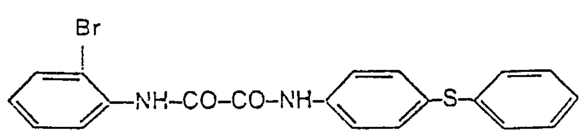 | 169–171 |

| Verbindung Nr. | | Smp. [°C] |
|---|---|---|
| 31 | 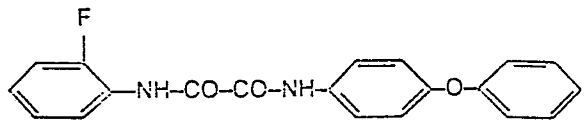 | 174–176 |
| 32 | 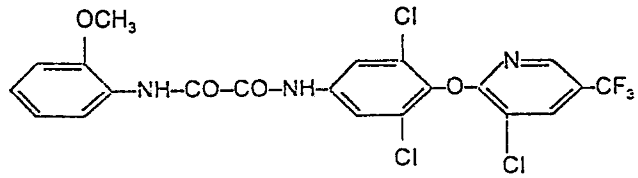 | 141–144 |
| 33 | 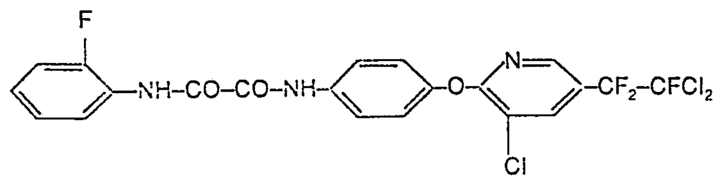 | 206–208 |
| 34 | 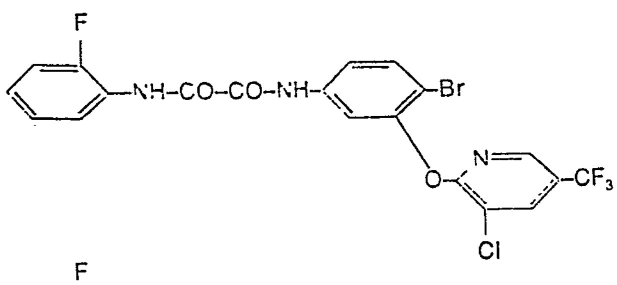 | 188–190 |
| 35 | 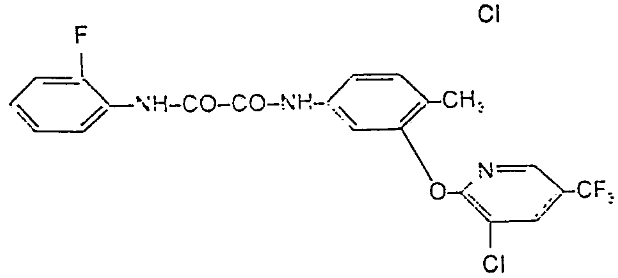 | 157–159 |
| 36 | 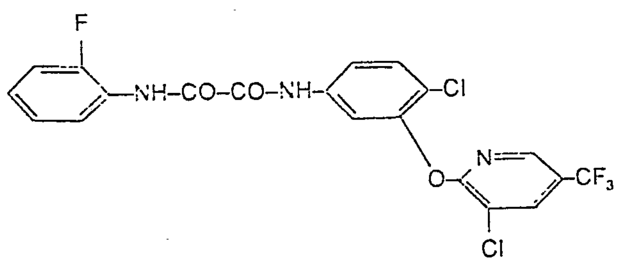 | 173–175 |
| 37 | 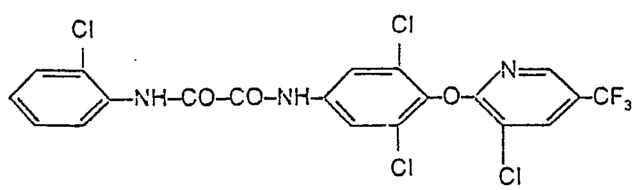 | 157–159 |
| 38 | 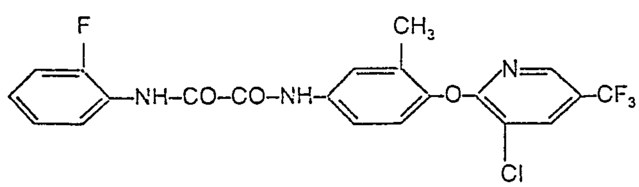 | 167–169 |

| Verbindung Nr. | | Smp. [°C] |
|---|---|---|
| 39 | 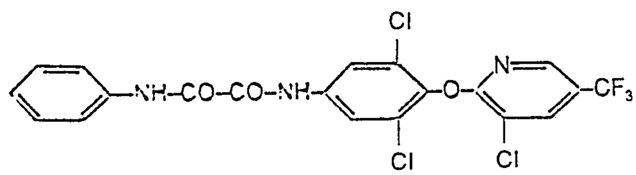 | 203–205 |
| 40 | 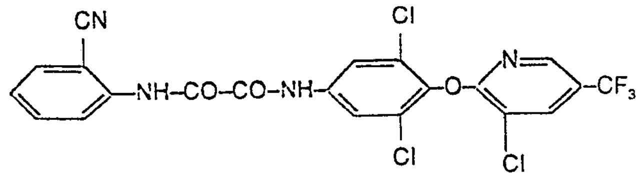 | 247–248 |
| 41 | 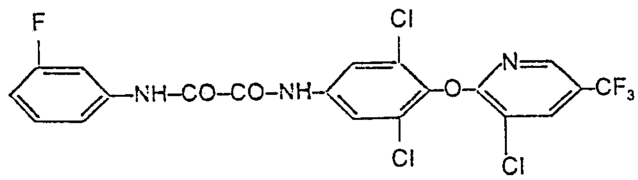 | 181–182 |
| 42 | 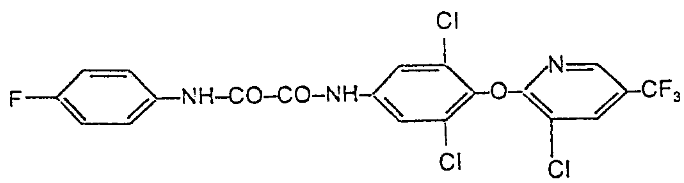 | 179–181 |
| 43 | 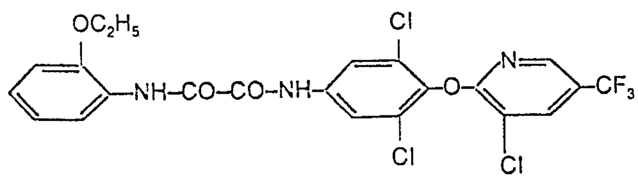 | 91–93 |
| 44 | 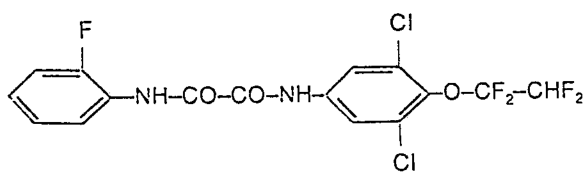 | 147–149 |
| 45 | 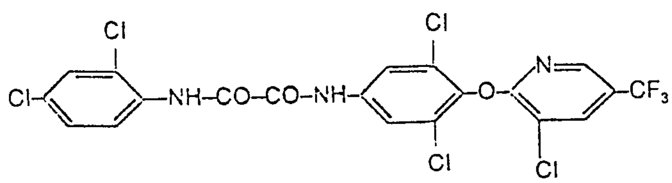 | 191–193 |
| 46 | 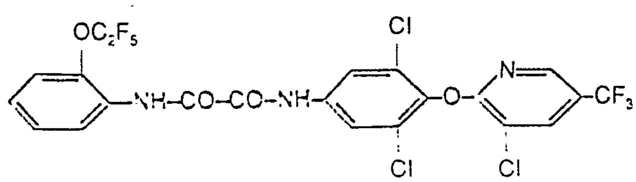 | 177–179 |
| 47 | 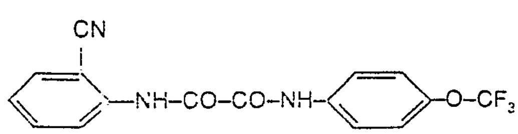 | 242–244 |

| Verbindung Nr. | | Smp. [°C] |
|---|---|---|
| 48 | 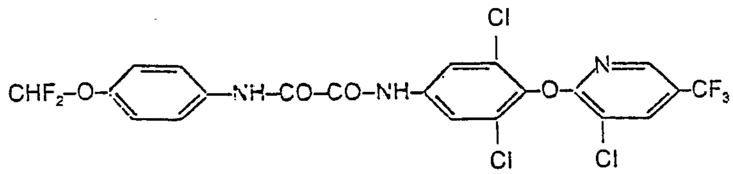 | 177–180 |
| 49 | 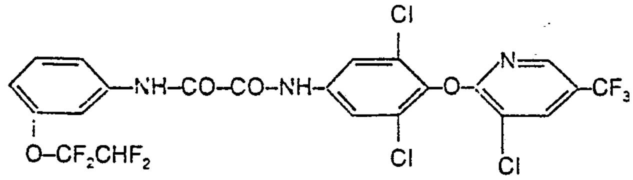 | 146–148 |
| 50 | 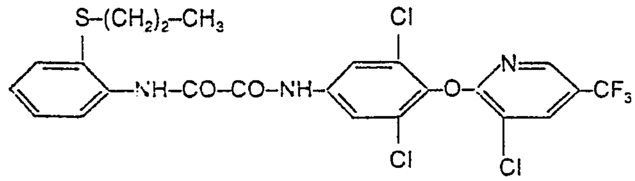 | 208–210 |
| 51 | 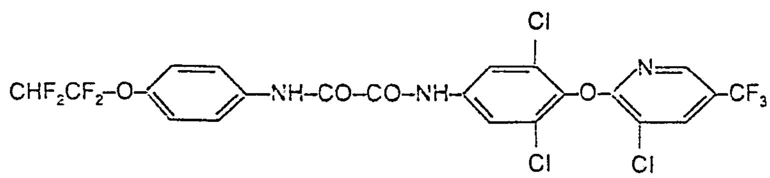 | 233–235 |
| 52 | 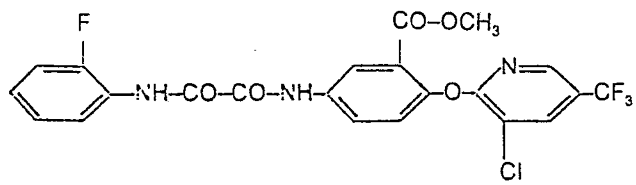 | 205–208 |
| 53 | 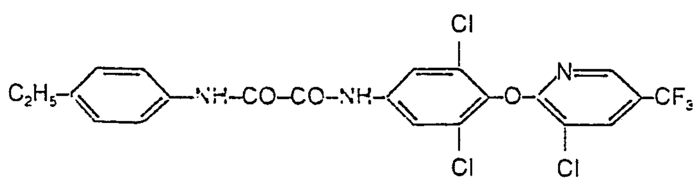 | 203–205 |
| 54 | 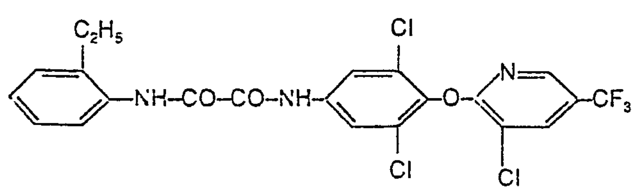 | 150–152 |
| 55 | 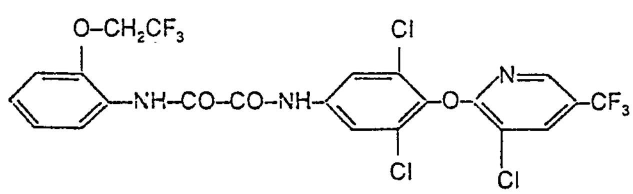 | 210–212 |
| 56 | 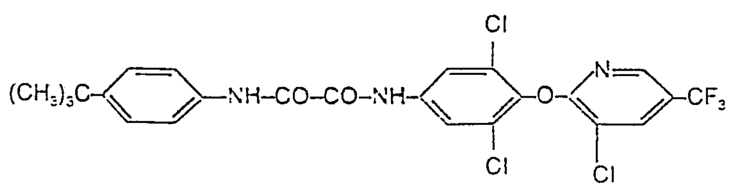 | 160 |

| Verbindung Nr. | | Smp. [°C] |
|---|---|---|
| 57 | 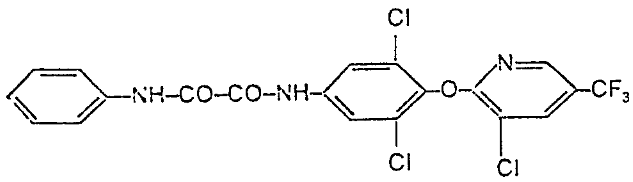 | 202–204 |
| 58 | 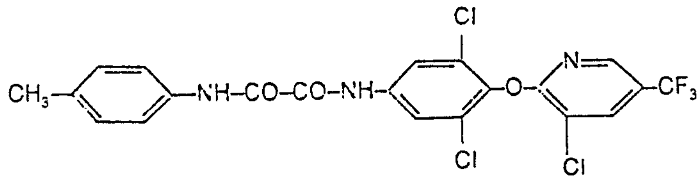 | 213–215 |
| 59 | 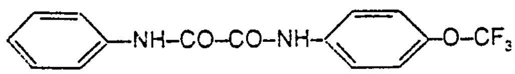 | 217–219 |
| 60 | 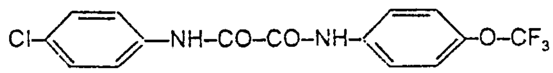 | 229–231 |
| 61 | 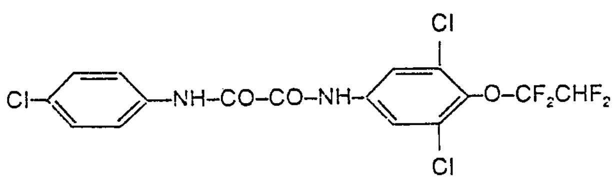 | 208–210 |
| 62 | 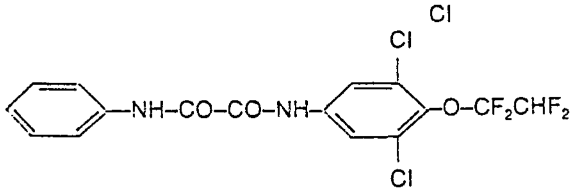 | 176–178 |
| 63 | 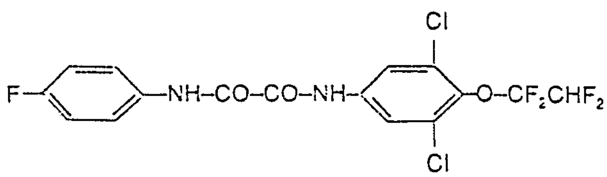 | 182–184 |
| 64 | 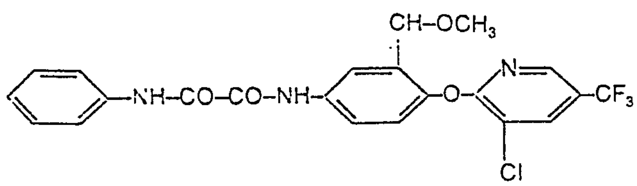 | 252–254 |
| 65 | 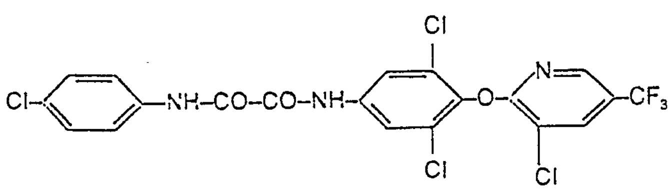 | 218–220 |
| 66 | 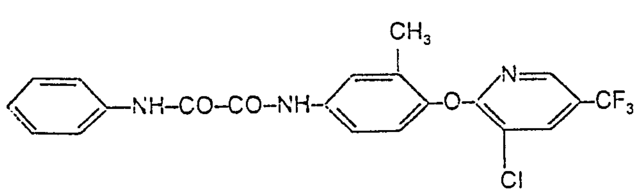 | 214–216 |

| Verbindung Nr. | | Smp. [°C] |
|---|---|---|
| 67 | 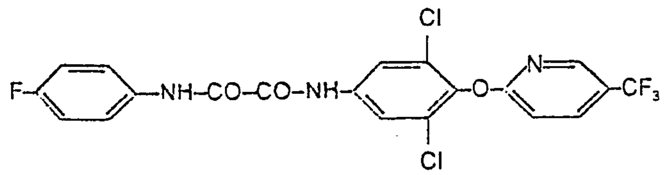 | 213–215 |
| 68 | 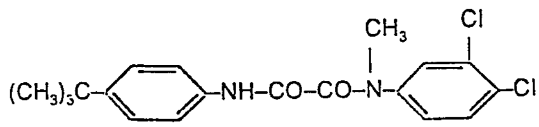 | 91–94 |
| 69 | 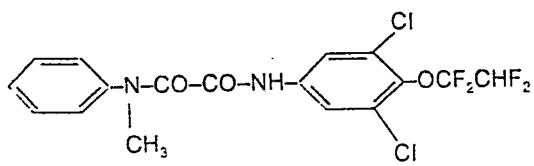 | 138–140 |
| 70 | 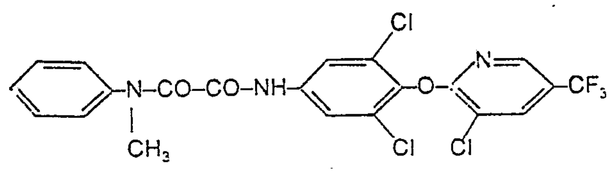 | 160–162 C° |

In entsprechender Weise wie vorstehend beschrieben sind auch die folgenden Verbindungen der Formel I herstellbar:

| Verbindung Nr. | |
|---|---|
| 71 | 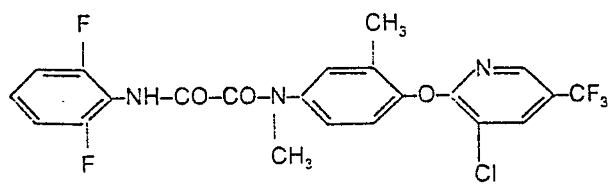 |
| 72 | 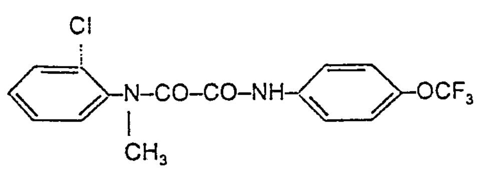 |
| 73 | 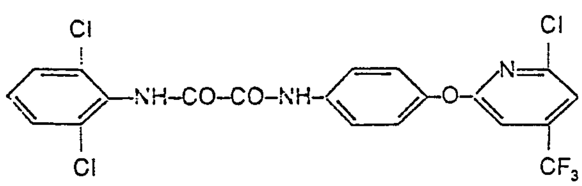 |

Beispiel 2

Wirkung gegen Musca domestica:

Je 50 g frisch zubereitetes Nährsubstrat für Maden wird in Becher eingewogen. Von einer 1 Gew.-%igen acetonischen Lösung des betreffenden Wirkstoffes wird eine bestimmte Menge auf das in den Bechern befindliche Nährsubstrat pipettiert, so daß sich eine Wirkstoffkonzentration von 400 ppm ergibt. Nach dem Durchmischen des Substrates läßt man das Aceton mindestens 20 Stunden lang verdampfen.

Dann werden pro Wirkstoff und Konzentration je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefäßen deponiert. Die pro Ansatz ausgeschwemmten Puppen werden gezählt (toxischer Einfluß des Wirkstoffes auf die Madenentwicklung). Dann wird nach 10 Tagen die Anzahl der aus den Puppen geschlüpften Fliegen bestimmt.

Verbindungen der Formel I gemäß Beispiel 1 zeigen gute Wirkung im obigen Test.

Beispiel 3
Wirkung gegen Lucilia sericata:

Zu 9 ml eines Zuchtmediums wird bei 50 °C 1 ml einer 0,5 Gew.-% Aktivsubstanz enthaltenden wässrigen Zubereitung hinzugefügt. Nun werden ca. 30 frisch geschlüpfte Lucilia sericata-Larven zum Zuchtmedium gegeben, und nach 48 und 96 Stunden wird die insektizide Wirkung durch Ermittlung der Abtötungsrate festgestellt.

Verbindungen der Formel I gemäss Beispiel 1 zeigen in diesem Test gute Wirkung gegen Lucilia sericata.

Beispiel 4
Wirkung gegen Aëdes aegypti:

Auf die oberfläche von 150 ml Wasser, das sich in einem Behälter befindet, wird so viel einer 0,1 Gew.-%igen acetonischen Lösung des Wirkstoffes pipettiert, daß eine Konzentration von 800 ppm erhalten wird. Nach Verdunsten des Acetons wird der Behälter mit 30 bis 40 2-tägigen Aëdes-Larven beschickt. Nach 1, 2 und 5 Tagen wird die Mortalität geprüft.

Verbindungen der Formel I gemäß Beispiel 1 zeigen in diesem Test gute Wirkung gegen Aëdes aegypti.

Beispiel 5
Insektizide Fraßgift-Wirkung:

Ca. 25 cm hohe eingetopfte Baumwollpflanzen werden mit wäßrigen Wirkstoffemulsionen besprüht, die den Wirkstoff in Konzentrationen von 400, 200, 50, 12,5 und 3,0 ppm enthalten.

Nach dem Antrocknen des Sprühbelages werden die Baumwollpflanzen mit Spodoptera littoralis- bzw. Heliothis virescens-Larven im dritten larvalen Stadium besiedelt. Der Versuch wird bei 24 °C und 60% relativer Luftfeuchtigkeit durchgeführt. Nach 120 Stunden wird die %-Mortalität der Test-Insekten bestimmt.

Beispiel 6
Wirkung gegen Epilachna varivestis:

Etwa 15–20 cm hohe Phaseolus vulgaris-Pflanzen (Buschbohnen) werden mit wäßrigen, den zu prüfenden Wirkstoff in Konzentrationen von 800 ppm enthaltenden Emulsions-Zubereitungen besprüht. Nach dem Antrocknen des Sprühbelages werden pro Pflanzen 5 Larven von Epilachna varivestis (Mexikanischer Bohnenkäfer) im 4. larvalen Stadium angesetzt. Über die infestierten Pflanzen wird ein Plastikzylinder gestülpt, der mit einem Kupfer-Gazedeckel abgedeckt ist. Der Versuch wird bei 28 °C und 60% relativer Luftfeuchtigkeit durchgeführt.

Nach 2 und 3 Tagen wird die % Mortalität bestimmt. Zur Auswertung hinsichtlich allfälligem Fraß-Schaden (Antifeeding-Effekt), Entwicklungs- und Häutungsstörungen werden die Versuchstiere während weiterer 3 Tage beobachtet.

Verbindungen der Formel I gemäß Beispiel 1 zeigen gute Wirkung im obigen Test.

Beispiel 7
Ovizide Wirkung auf Heliothis virescens:

Entsprechende Mengenanteile einer benetzbaren pulverförmigen Formulierung, enthaltend 25 Gew.-% des zu prüfenden Wirkstoffes, werden mit jeweils soviel Wasser vermischt, daß sich eine wäßrige Emulsion mit einer Wirkstoffkonzentration von 800 ppm ergibt.

In diese wirkstoffhaltige Emulsionen werden eintägige Eigelege von Heliothis auf Cellophan während drei Minuten eingetaucht und dann auf Rundfilter abgenutscht. Die so behandelten Gelege werden in Petrischalen ausgelegt und in der Dunkelheit aufbewahrt. Nach 6 bis 8 Tagen wird die Schlupfrate im Vergleich zu unbehandelten Kontrollen festgestellt. Zur Auswertung wird die zur 100%igen Abtötung der Eier erforderliche minimale Wirkstoffkonzentration bestimmt.

Verbindungen der Formel I gemäß Beispiel 1 zeigen gute Wirkung in obigem Test.

Beispiel 8
Wirkung auf Laspeyresia pomonella (Eier):

Abgelegte Eier von Laspeyresia pomonella, die nicht älter als 24 Stunden sind, werden auf Filterpapier für 1 Minute in eine acetonisch-wäßrige Lösung, enthaltend 800 ppm des zu prüfenden Wirkstoffes, eingetaucht. Nach dem Antrocknen der Lösung werden die Eier in Petrischalen ausgelegt und bei einer Temperatur von 28 °C belassen. Nach 6 Tagen wird der prozentuale Schlupf aus den behandelten Eiern bewertet und die %-Mortalität bestimmt.

Verbindungen der Formel I gemäß Beispiel 1 zeigen gute Wirkung in obigem Test.

Beispiel 9
Reproduktions-Beeinflussung von Anthonomus grandis:

Adulte Anthonomus grandis, die nach dem Schlupf nicht älter als 24 Stunden waren, werden in Gruppen zu jeweils 25 Käfern in Käfige mit Gitterwänden überführt. Die mit den Käfern besetzten Käfige werden sodann während 5 bis 10 Sekunden in eine acetonische Lösung, enthaltend 1,0 Gew.-% des zu prüfenden Wirkstoffes, eingetaucht. Nachdem die Käfer wieder trocken sind, werden sie zur Kopulation und Eiablage in abgedeckte und Futter enthaltende Schalen eingesetzt. Abgelegte Eier werden zwei- bis dreimal wöchentlich mit fließendem Wasser ausgeschwemmt, gezählt, durch zwei- bis dreistündiges Einlegen in ein wäßriges Desinfektionsmittel desinfiziert und dann in Schalen, die eine geeignete Larvaldiät enthalten, deponiert. Nach 7 Tagen wird untersucht, ob sich aus den deponierten Eiern Larven entwickelt haben.

Zur Ermittlung der Dauer des die Reproduktion beeinflussenden Effektes der zu prüfenden Wirkstoffe wird die Eiablage der Käfer während eines Zeitraums von etwa 4 Wochen überprüft. Die Bonitierung erfolgt anhand der Verminderung der Anzahl abgelegter Eier und der daraus geschlüpften Larven im Vergleich zu unbehandelten Kontrollen.

Verbindungen der Formel I gemäß Beispiel 1 zeigen eine gute reproduktionsreduzierende Wirkung im obigen Test.

Beispiel 10
Akarizide Wirkung:

Phaseolus vulgaris Pflanzen werden 12 Stunden vor dem Test auf akarizide Wirkung mit einem infestierten Blattstück aus einer Massenzucht von Tetranychus urticae belegt. Die übergelaufenen beweglichen Stadien werden aus einem Chromatographiezerstäuber mit den emulgierten Testpräparaten derart besprüht, daß kein Ablaufen der Spritzbrühe eintritt. Die jeweils verwendeten Emulsionszubereitungen weisen eine Wirkstoffkonzentration von 800 ppm auf. Nach zwei und 7 Tagen werden Larven, Adulte und Eier unter dem Binokular auf lebende und tote Individuen ausgewertet; das Ergebnis wird in Prozenten ausgedrückt.

Während der «Haltezeit» stehen die behandelten Pflanzen in Gewächshauskabinen bei 25 °C.

Verbindungen der Formel I gemäß Beispiel 1 zeigen im obigen Test gute Wirkung.

Beispiel 11
Wirkung gegen Anthonomus grandis (Adulte)

Zwei eingetopfte Baumwollpflanzen im 6-Blattstadium werden jeweils mit einer wäßrigen benetzungsfähigen Emulsions-Zubereitung, enthaltend 50 bzw. 200 ppm des zu prüfenden Wirkstoffes besprüht. Nach dem Antrocknen des Spritzbelages (nach etwa 1,5 Stunden) wird jede Pflanze mit 10 adulten Käfern (Anthonomus grandis) besiedelt. Plastikzylinder, deren obere Öffnungen mit Gaze abgedeckt sind, werden dann über die behandelten, mit den Testtieren besiedelten Pflanzen gestülpt, um ein Abwandern der Käfer zu verhindern. Die so behandelten Pflanzen werden bei 25 °C und etwa 60% relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie der Antifeedant-Wirkung gegenüber unbehandelten Kontrollansätzen.

Biologische Ergebnisse

Die nachstehende Tabelle zeigt Ergebnisse biologischer Prüfungen erfindungsgemäßer Verbindungen auf der Basis der obigen biologischen Beispiele. Die Auswertung der Versuche erfolgt anhand der erhaltenen %-Mortalität unter Verwendung des folgenden Bewertungs-Index:

A: 80–100% Mortalität bei einer Konzentration von 3,0 ppm der geprüften Verbindung.
B: 80–100% Mortalität bei einer Konzentration von 12,5 ppm der geprüften Verbindung.
C: 80–100% Mortalität bei einer Konzentration von 50 ppm der geprüften Verbindung.
D: 80–100% Mortalität bei einer Konzentration von 100 ppm der geprüften Verbindung.
E: 80–100% Mortalität bei einer Konzentration von 200 ppm der geprüften Verbindung.
F: 80–100% Mortalität bei einer Konzentration von 400 ppm der geprüften Verbindung;
–: nicht geprüft.

| Verbindung Nr. | pestizide Wirksamkeit | | |
|---|---|---|---|
| | Spodoptera (Beispiel 5) | Heliothis (Beispiel 5) | Anthonomus (Beispiel 11) |
| 1 | B | C | E |
| 4 | F | – | C |
| 10 | A | B | C |
| 13 | F | – | – |
| 17 | F | – | – |
| 18 | C | – | – |
| 20 | E | – | – |
| 23 | F | – | – |
| 24 | F | – | – |
| 27 | C | D | E |
| 28 | C | F | F |
| 32 | F | – | – |
| 35 | F | – | – |
| 39 | A | E | – |
| 40 | D | – | – |
| 41 | F | – | – |
| 42 | B | F | – |
| 43 | F | – | – |
| 44 | F | – | – |
| 45 | F | – | – |

**Patentansprüche**

1. Schädlingsbekämpfungsmittel, enthaltend als aktive Komponente eine Verbindung der Formel I

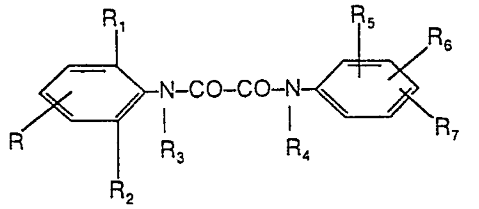

(I),

worin

R Wasserstoff, Halogen, $C_1$–$C_4$-Alkyl, Methoxy, Äthoxy, $C_1$–$C_2$-Fluoralkoxy mit 1 bis 5 Fluoratomen;

$R_1$ Wasserstoff, Halogen, Methyl, Methoxy, Äthoxy, $C_1$–$C_2$-Fluoralkoxy mit 1 bis 5 Fluoratomen, $C_1$–$C_3$-Alkylthio oder Cyano;

$R_2$ Wasserstoff, Halogen, Methyl oder Methoxy;

$R_3$ und $R_4$ unabhängig voneinander Wasserstoff oder Methyl;

$R_5$ Wasserstoff, Halogen, Methyl, Acetyl oder Trifluormethyl;

$R_6$ Wasserstoff, Halogen, Methyl, Trifluormethyl oder Carbalkoxy mit 1 bis 4 Kohlenstoffatomen im Alkylteil; und

$R_7$ Wasserstoff, $C_1$–$C_3$-Fluoralkyl mit 1 bis 7 Fluoratomen, $C_1$–$C^3$-Alkoxy, $C_1$–$C_3$-Halogenalkoxy mit 1 bis 7 Halogenatomen, oder den Rest

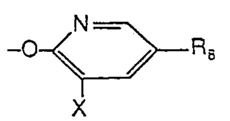

,

wobei $R_8$ für Wasserstoff, Chlor, Trifluormethyl oder einen perhalogenierten Äthylrest und X für Wasserstoff oder Chlor stehen, oder den Rest

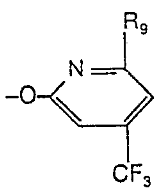

,

wobei $R_9$ für Wasserstoff, Chlor, Brom, Methoxy oder Äthoxy steht, oder den Rest

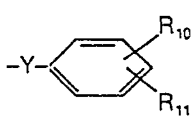

wobei $R_{10}$ und $R_{11}$ unabhängig voneinander für Wasserstoff, Halogen, Methyl, Trifluormethoxy oder Trifluormethyl und Y für ein Sauerstoff- oder Schwefelatom stehen, oder den Rest

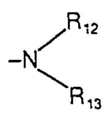

,

wobei $R_{12}$ und $R_{13}$ für $C_1$–$C_5$-Alkyl stehen, bedeuten, mit der Maßgabe, daß $R_5$, $R_6$ und $R_7$ nicht gleichzeitig Wasserstoff bedeuten, zusammen mit geeigneten Trägern und/oder anderen Zuschlagstoffen.

2. Verbindung der Formel Ia

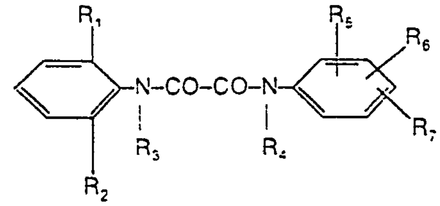

(Ia),

worin

$R_1$ Wasserstoff, Fluor, Chlor oder Methoxy;

$R_2$ Wasserstoff, Fluor, Chlor, Brom oder Methoxy;

$R_3$ und $R_4$ unabhängig voneinander Wasserstoff oder Methyl;

$R_5$ und $R_6$ unabhängig voneinander Wasserstoff, Chlor oder Methyl;

$R_7$ Trifluormethoxy, dem Rest $-O-CF_2-CHF_2$ oder den Rest

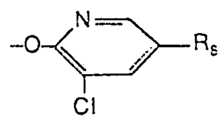

,

wobei $R_8$ für Trifluormethyl oder einen der Reste $-CF_2-CF_2Cl$, $-CF_2-CF\ Cl_2$, $-CCl_2-CCl_3$, $-CF_2-CCl_3$ oder $-CF_2-CF_3$ steht, oder den Rest

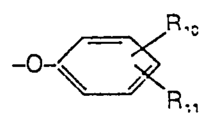

,

wobei $R_{10}$ und $R_{11}$ unabhängig voneinander für Wasserstoff oder Chlor stehen, oder den Rest

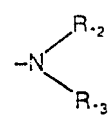

,

wobei $R_{12}$ für Methyl und $R_{13}$ für $C_1$–$C_5$-Alkyl stehen;

bedeuten.

3. Verbindung der Formel I gemäß Anspruch 2, dadurch gekennzeichnet, daß $R_7$ in 4-Stellung am Phenylring steht.

4. Verbindung der Formel I gemäß Anspruch 2, dadurch gekennzeichnet, daß $R_5$ Wasserstoff bedeutet und $R_7$ in 5-Stellung am Phenylring steht.

5. Verbindung der Formel I gemäß Anspruch 4, dadurch gekennzeichnet, daß $R_6$ in 4-Stellung am Phenylring steht.

6. Verbindung der Formel I gemäß Anspruch 2, dadurch gekennzeichnet, daß $R_5$ und $R_6$ in 3- bzw. 5-Stellung am Phenylring stehen.

7. Verbindung der Formel I gemäß Anspruch 3, dadurch gekennzeichnet, daß $R_7$ einen in 4-Stellung befindlichen Rest aus der Gruppe

$-O-CF_2CHF_2$ , 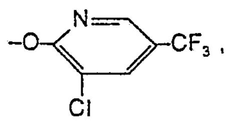 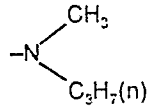 oder 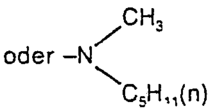

bedeutet.

8. Verbindung gemäß Anspruch 7 der Formel

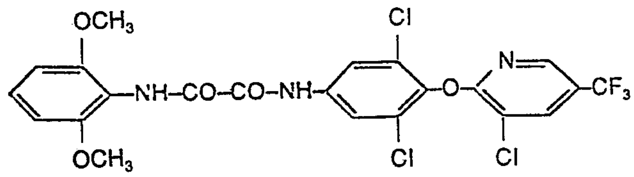

9. Verbindung gemäß Anspruch 7 der Formel

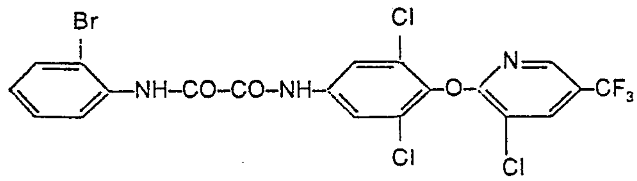

10. Verbindung gemäß Anspruch 7 der Formel

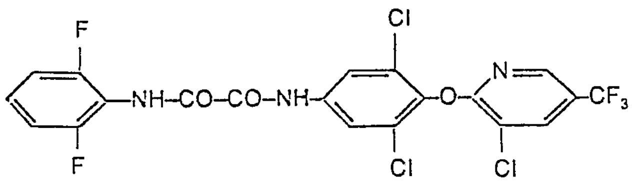

11. Verbindung gemäß Anspruch 2 der Formel

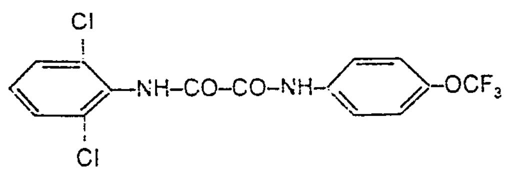

12. Verbindung gemäß Anspruch 7 der Formel

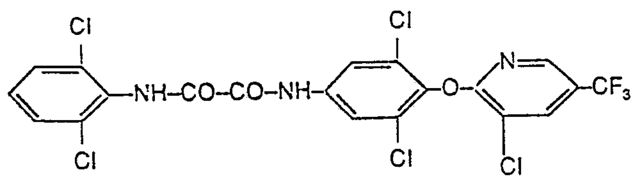

13. Verbindung gemäß Anspruch 7 der Formel

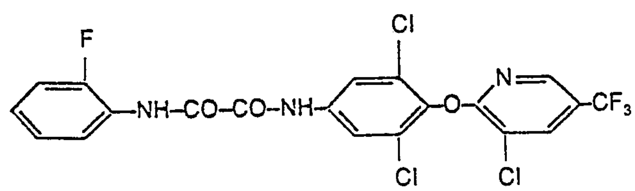

14. Verbindung gemäß Anspruch 2 der Formel

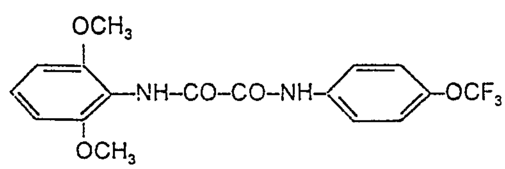

15. Verbindung gemäß Anspruch 7 der Formel

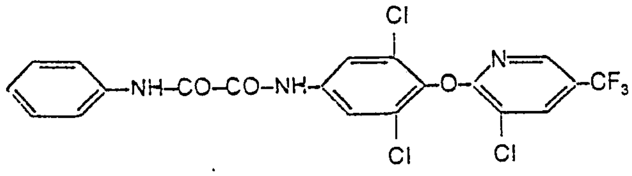

16. Verbindung gemäß Anspruch 7 der Formel

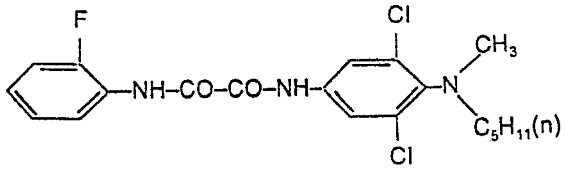

17. Verfahren zur Herstellung einer Verbindung der Formel Ia gemäß einem der Ansprüche 2 bis 16, dadurch gekennzeichnet, daß man

a) eine Verbindung der Formel II

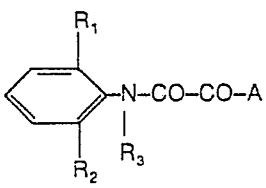

(II)

mit einer Verbindung der Formel III

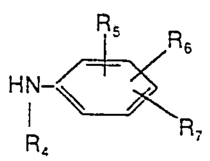

(III)

oder

b) eine Verbindung der Formel IV

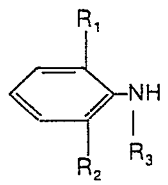

(IV)

mit einer Verbindung der Formel V

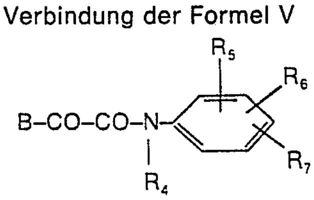

(V)

umsetzt, wobei in den Formeln II bis V die Reste $R_1$ bis $R_7$ die in den Ansprüchen 1 bis 7 angegebenen Bedeutungen haben, A Methoxy, Äthoxy oder Halogen, vorzugsweise Chlor, und B Methoxy oder Äthoxy bedeuten.

18. Verwendung einer Verbindung der Formel I gemäß einem der Ansprüche 1 bis 16 zur Bekämpfung von Insekten.

19. Verwendung gemäß Anspruch 18 zur Bekämpfung larvaler Stadien pflanzenschädigender Insekten.

**Claims**

1. A pesticidal composition containing as active component a compound of formula I

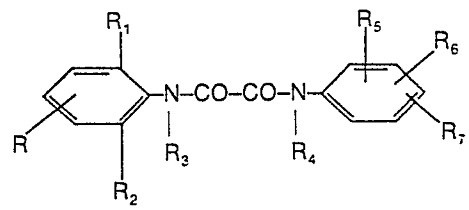

(I),

wherein

R is hydrogen, halogen, $C_1$–$C_4$alkyl, methoxy, ethoxy, $C_1$–$C_2$fluoroalkoxy containing 1 to 5 fluorine atoms,

$R_1$ is hydrogen, halogen, methyl, methoxy, ethoxy, $C_1$–$C_2$fluoroalkoxy containing 1 to 5 fluorine atoms, $C_1$–$C_3$alkylthio or cyano,

$R_2$ is hydrogen, halogen, methyl or methoxy,

$R_3$ and $R_4$ are each independently hydrogen or methyl,

$R_5$ is hydrogen, halogen, methyl, acetyl or trifluoromethyl,

$R_6$ is hydrogen, halogen, methyl, trifluoromethyl or carbalkoxy containing 1 to 4 carbon atoms in the alkyl moiety, and

$R_7$ is hydrogen, $C_1$–$C_3$fluoroalkyl containing 1 to 7 fluorine atoms, $C_1$–$C_3$alkoxy, $C_1$–$C_3$haloalkoxy containing 1 to 7 halogen atoms or is the

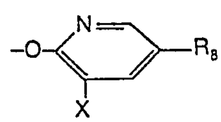

,

radical, in which $R_8$ is hydrogen, chlorine, trifluoromethyl or a perhalogenated ethyl radical and X is hydrogen or chlorine; or is the

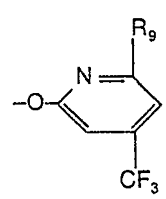

,

radical, in which $R_9$ is hydrogen, chlorine, bromine, methoxy or ethoxy; or is the

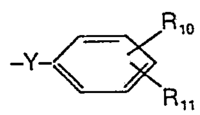

radical, in which $R_{10}$ and $R_{11}$ are each independently of the other hydrogen, halogen, methyl, trifluoromethoxy or trifluoromethyl and Y is an oxygen or a sulfur atom; or is the

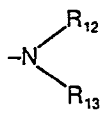,

radical, in which $R_{12}$ and $R_{13}$ are $C_1$–$C_5$alkyl, with the proviso that $R_5$, $R_6$ and $R_7$ may not simultaneously be hydrogen, together with suitable carriers and/or other adjuvants.

2. A compound of formula Ia,

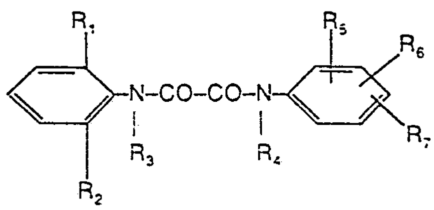

(Ia),

wherein

$R_1$ is hydrogen, fluorine, chlorine or methoxy,

$R_2$ is hydrogen, fluorine, chlorine, bromine or methoxy

$R_3$ and $R_4$ are each independently of the other hydrogen or methyl,

$R_5$ and $R_6$ are each independently of the other hydrogen, chlorine or methyl,

$R_7$ is trifluoromethoxy, the $-O-CF_2-CHF_2$ radical or the

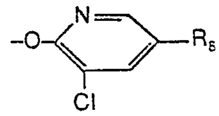,

radical, in which $R_8$ is trifluoromethyl or radical selected from the group consisting of $-CF_2-CF_2Cl$, $-CF_2CFCl_2$, $-CCl_2-CCl_3$, $-CF_2CCl_3$ or $-CF_2-CF_3$, or the

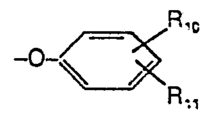,

radical, in which $R_{10}$ and $R_{11}$ are each independently of the other hydrogen or chlorine, or the

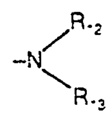,

radical, in which $R_{12}$ is methyl and $R_{13}$ is $C_1$–$C_5$alkyl.

3. A compound of formula I according to claim 2, wherein $R_7$ is in the 4-position on the phenyl ring.

4. A compound of formula I according to claim 2, wherein $R_5$ is hydrogen and $R_7$ is in the 5-position on the phenyl ring.

5. A compound of formula I according to claim 4, wherein $R_6$ is in the 4-position on the phenyl ring.

6. A compound of formula I according to claim 2, wherein $R_5$ and $R_6$ are in the 3- and 5-position respectively on the phenyl ring.

7. A compound of formula I according to claim 3, wherein $R_7$ a radical selected from the group consisting of

$-O-CF_2CHF_2$, 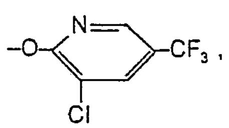, 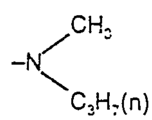 or 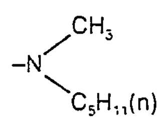 said radical being in the 4-position.

8. A compound according to claim 7 of formula

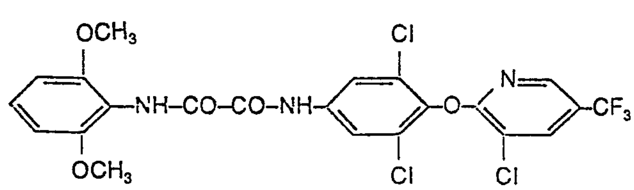.

9. A compound according to claim 10 of formula

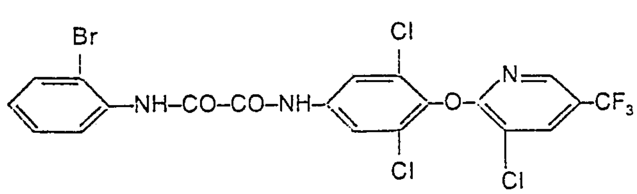

10. A compound according to claim 7 of formula

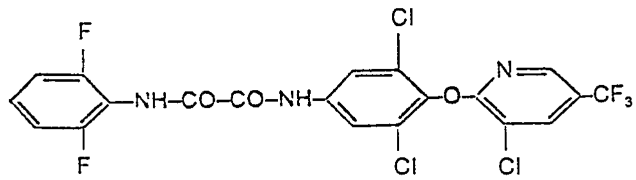

11. A compound according to claim 2 of formula

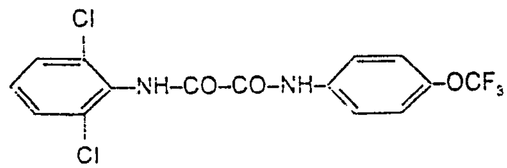

12. A compound according to claim 7 of formula

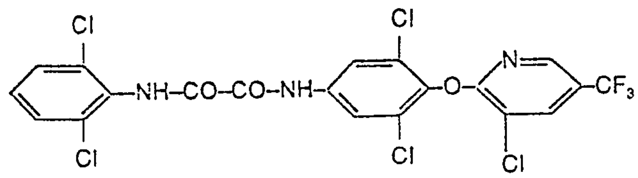

13. A compound according to claim 7 of formula

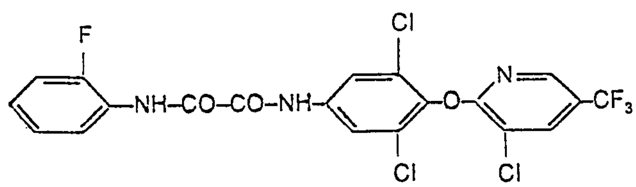

14. A compound according to claim 2 of formula

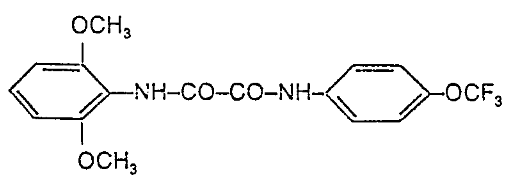

15. A compound according to claim 7 of formula

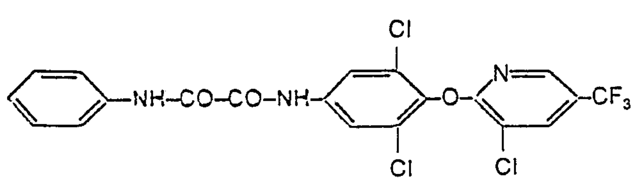

16. A compound according to claim 7 of formula

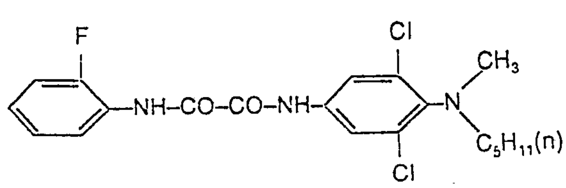

17. A process for the preparation of a compound of formula Ia according to any one of claims 2 to 16, which comprises

a) reacting a compound of the formula II

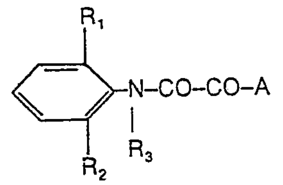

(II)

with a compound of formula III

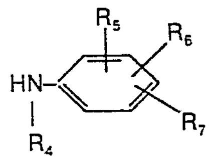

(III)

or

b) reacting a compound of formula IV

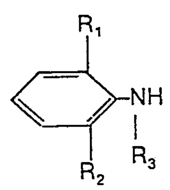

(IV)

with a compound of formula V

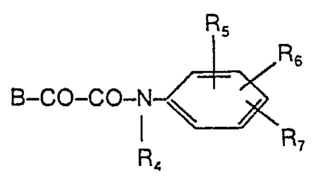

(V)

in which formula II to V above the symbols $R_1$ to $R_7$ are as defined in claims 2 to 7, A is methoxy, ethoxy or halogen, preferably chlorine, and B is methoxy or ethoxy.

18. Use of a compound of formula I according to any one of claims 1 to 16 for controlling insects.

19. Use according to claim 18 for controlling larval stages of plantdestructive insects.

**Revendications**

1. Produit pesticide contenant en tant que composant actif un composé de formule I

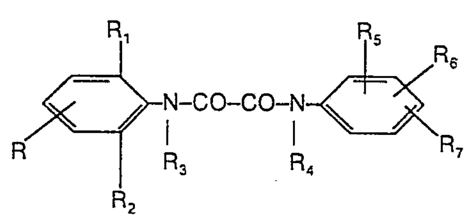

(I),

dans laquelle

R représente l'hydrogène, un halogène, un groupe alkyle en C 1–C 4, méthoxy, éthoxy, fluoralcoxy contenant 1 ou 2 atomes de carbone et 1 à 5 atomes de fluor,

$R_1$ représente l'hydrogéne, un halogène, un groupe méthyle, méthoxy, éthoxy, fluoralcoxy contenant 1 ou 2 atomes de carbone et 1 à 5 atomes de fluor, alkylthio en C 1–C 3 ou cyano,

$R_2$ représente l'hydrogène, un halogène, un groupe méthyle ou méthoxy,

$R_3$ et $R_4$ représentent chacun, indépendamment l'un de l'autre, l'hydrogène ou un groupe méthyle,

$R_5$ représente l'hydrogène, un halogène, un groupe méthyle, acétyle ou trifluorométhyle,

$R_6$ représente l'hydrogène, un halogène, un groupe méthyle, trifluorométhyle ou carbalcoxy contenant 1 à 4 atomes de carbone dans la partie alkyle; et

$R_7$ représente l'hydrogène, un groupe fluoralkyle contenant 1 à 3 atomes de carbone et 1 à 7 atomes de fluor, un groupe alcoxy en C 1–C 3, halogénoalcoxy contenant 1 à 3 atomes de carbone et 1 à 7 atomes d'halogènes, ou le groupe

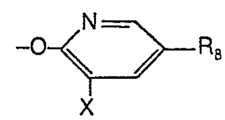

.

dans lequel $R_8$ représente l'hydrogène, le chlore, un groupe trifluorométhyle ou un groupe éthyle perhalogéné et X représente l'hydrogène ou le chlore, ou le groupe

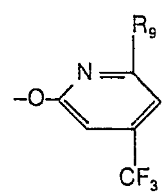

.

dans lequel $R_9$ représente l'hydrogène, le chlore, le brome, un groupe méthoxy ou éthoxy, ou le groupe

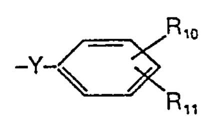

dans lequel $R_{10}$ et $R_{11}$ représentent chacun, indépendamment l'un de l'autre, l'hydrogène, un halogène, un groupe méthyle, trifluorométhoxy ou trifluorométhyle et Y représente un atome d'oxygène ou de soufre, ou le groupe

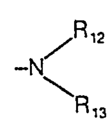

,

dans lequel $R_{12}$ et $R_{13}$ représentent des groupes alkyle en C 1–C 5, étant spécifié toutefois que $R_5$, $R_6$ et $R_7$ ne peuvent représenter tous simultanément l'hydrogène, avec des véhicules et/ou d'autres additifs appropriés.

2. Composé de formule Ia

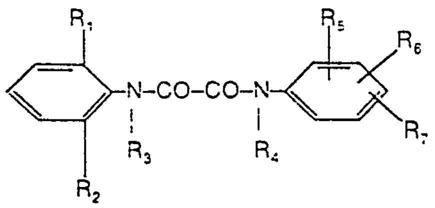

(Ia),

dans laquelle

$R_1$ représente l'hydrogène, le fluor, le chlore ou un groupe méthoxy;

$R_2$ représente l'hydrogène, le fluor, le chlore, le brome ou un groupe méthoxy;

$R_3$ et $R_4$ représentent chacun, indépendamment l'un de l'autre, l'hydrogène ou un groupe méthyle;

$R_5$ et $R_6$ représentent chacun, indépendamment l'un de l'autre, l'hydrogène, le chlore ou un groupe méthyle;

$R_7$ représente un groupe trifluorométhoxy, le groupe $-O-CF_2-CHF_2$ ou le groupe

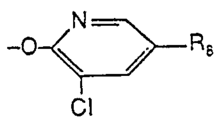

,

dans lequel $R_8$ représente un groupe trifluorométhyle ou l'un des groupes $-CF_2-CF_2Cl$, $-CF_2-CFCl_2$, $CCl_2-CCl_3$, $-CF_2-CCl_3$ ou $-CF_2-CF_3$, ou le groupe

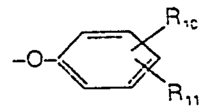

,

dans lequel $R_{10}$ et $R_{11}$ représentent chacun, indépendamment l'un de l'autre, l'hydrogène ou le chlore, ou le groupe

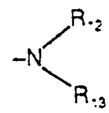

,

dans lequel $R_{12}$ représente un groupe méthyle et $R_{13}$ un groupe alkyle en C 1–C 5.

3. Composé de formule I selon la revendication 2, caractérisé en ce que $R_7$ est en position 4 du noyau phényle.

4. Composé de formule I selon la revendication 2, caractérisé en ce que $R_5$ représente l'hydrogène et $R_7$ est en position 5 du noyau phényle.

5. Composé de formule I selon la revendication 4, caractérisé en ce que $R_6$ est en position 4 du noyau phényle.

6. Composé de formule I selon la revendication 2, caractérisé en ce que $R_5$ et $R_6$ sont respectivement dans les positions 3 et 5 du noyau phényle.

7. Composé de formule I selon la revendication 3, caractérisé en ce que $R_7$ représente un groupe placé en position 4 et choisi parmi les suivants:

$-O-CF_2CHF_2$ ,

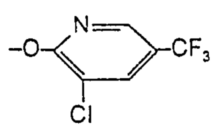

,

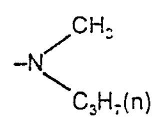

ou

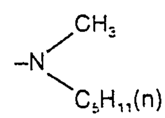

8. Composé selon la revendication 7, de formule

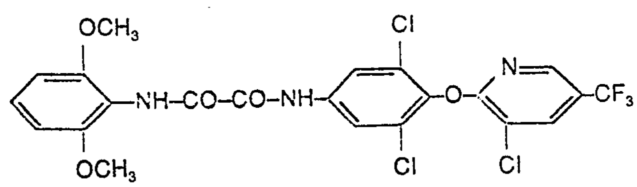

9. Composé selon la revendication 7, de formule

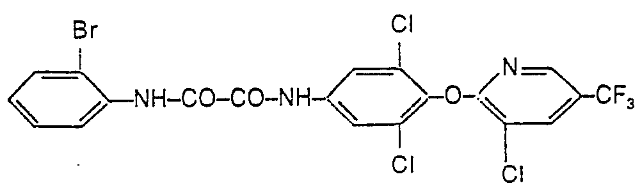

10. Composé selon la revendication 7, de formule

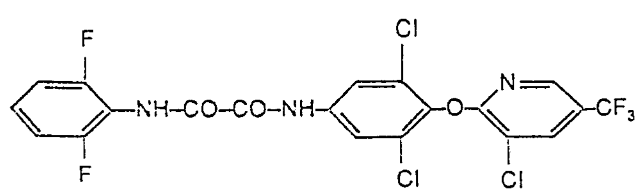

11. Composé selon la revendication 2, de formule

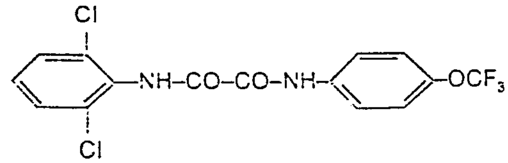

12. Composé selon la revendication 7, de formule

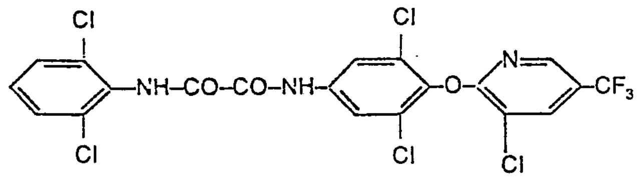

13. Composé selon la revendication 7, de formule

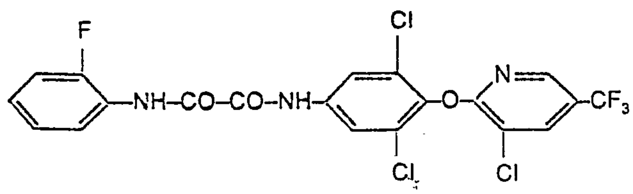

14. Composé selon la revendication 2, de formule

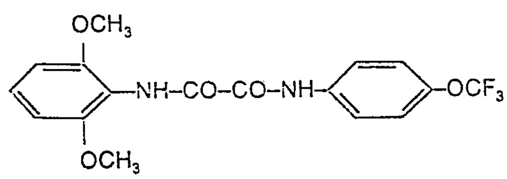

15. Composé selon la revendication 7, de formule

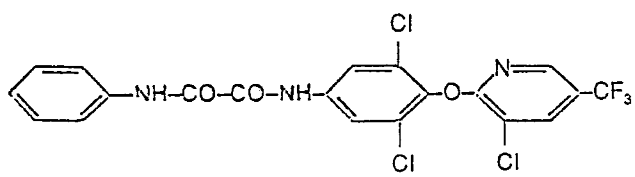

16. Composé selon la revendication 7, de formule

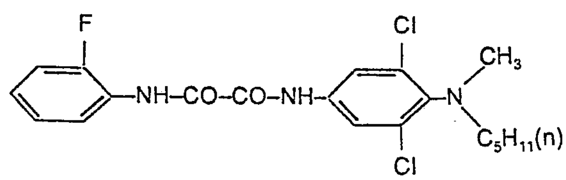

17. Procédé de préparation d'un composé de formule Ia selon l'une des revendications 2 à 16, caractérisé en ce que:

a) on fait réagir un composé de formule II

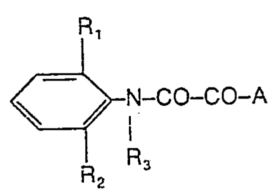

(II)

avec un composé de formule III

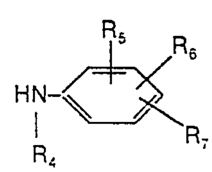

(III)

ou bien

b) on fait réagir un composé de formule IV

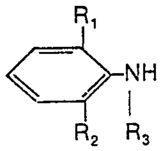

(IV)

avec un composé de formule V

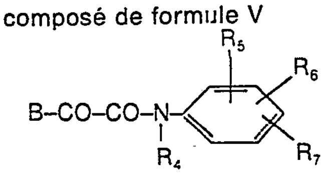

(V)

les symboles $R_1$ à $R_7$ ayant dans les formules II à V les significations indiquées dans les revendications 2 à 7, A représente un groupe méthoxy, éthoxy ou un halogène, de préférence le chlore, et B un groupe méthoxy ou éthoxy.

18. Utilisation d'un composé de formule I selon l'une des revendications 1 à 16 dans la lutte contre les insectes.

19. Utilisation selon la revendication 18 dans la lutte contre les stades larvaires des insectes nuisible pour les végétaux.